# FASCICULE DE BREVET EUROPEEN

(11) **EP 2 892 491 B1**
(45) Date de publication et mention de la délivrance du brevet: **16.11.2016**
(21) Numéro de dépôt: 13756410.0
(22) Date de dépôt: 27.08.2013
(51) Int. Cl.: A61G 9/00, A61F 5/44

(54) **POCHE SOUPLE A MANCHON D'OUVERTURE**
FLEXIBLER BEUTEL MIT EINER HÜLSE ZUM ÖFFNEN
FLEXIBLE POUCH HAVING A SLEEVE FOR OPENING

(30) Priorité: 07.09.2012 FR 1258388
(43) Date de publication de la demande: 15.07.2015
(73) Titulaire: M3AT SA, 1652 Botterens (CH)
(72) Inventeur: CAILLETEAU, Benoît, F-13007 Marseille (FR)
(74) Mandataire: Intès, Didier Gérard André
(86) Numéro de dépôt international: PCT/EP2013/067712
(87) Numéro de publication internationale: WO 2014/037246

(56) Documents cités:
- EP-A1- 0 847 742
- FR-A1- 2 759 981
- US-A- 5 065 459
- US-B1- 6 345 911

## Description

La présente invention concerne une poche comprenant une enveloppe souple ayant une ouverture dont le bord est fixé aux deux parois opposées d'un manchon de renfort en saillie à l'extérieur de l'enveloppe, la poche étant susceptible d'adopter une configuration aplatie dans laquelle lesdites parois sont situées l'une contre l'autre, et une configuration d'utilisation dans laquelle ces parois forment un canal qui maintient l'ouverture ouverte.

Des poches de ce type sont connues, par exemple par le brevet européen n° 0 847 742. Elles sont utilisées pour recevoir des produits sous forme généralement liquide, notamment des déchets d'origine humaine ou animale tels que de l'urine. Ces poches sont stockées dans leur configuration aplatie. Pour leur utilisation, le manchon est manipulé de manière à ce que ses parois forment le canal qui maintient l'ouverture ouverte, mettant ainsi la poche dans sa configuration d'utilisation, dans laquelle les déchets peuvent être déversés à l'intérieur de cette poche. Une fois que les déchets ont été introduits, il suffit de relâcher le manchon pour que la poche reprenne sa configuration aplatie.

Ces poches sont couramment réalisées au moyen de feuilles minces de matière plastique ou autre matériaux, éventuellement transparentes, dotées de la souplesse voulue. Pour l'ouverture, la manipulation du manchon consiste à déformer ses parois de manière à les contraindre à adopter une forme convexe (vue de l'extérieur de la poche) dans laquelle elles forment entre elles le canal qui maintient l'ouverture ouverte.

Les dimensions transversales de l'ouverture sont choisies de manière appropriée à l'usage que l'on souhaite faire de la poche. Généralement, notamment lorsque la poche est destinée à recevoir des urines, le manchon doit pouvoir être manipulé d'une seule main, et ses dimensions transversales sont alors en général nettement inférieures aux dimensions transversales courantes de la poche.

La demande de brevet européen n° 0 847 742 s'intéresse au fait d'assurer que la manipulation du manchon le conformant en canal provoque l'écartement des parois de l'enveloppe, non seulement sur les bords de l'ouverture de l'enveloppe, mais également en aval de cette ouverture, pour éviter que le produit introduit dans la poche n'ait tendance à refluer. Pour cela, le manchon est doté de pattes internes d'écartement. Ceci donne une grande liberté de choix quant au matériau constituant l'enveloppe de la poche. En effet, grâce à l'invention objet de la demande de brevet européen précitée, on assure que les feuilles de l'enveloppe s'écartent même si elles sont très souples, voire si elles ont initialement une tendance à rester légèrement collées.

Un autre problème posé à l'élaboration de poche du type précité est celui d'assurer une conduction efficace des produits (en particulier des déchets tels que de l'urine) à l'intérieur de l'enveloppe. Lorsque la poche est utilisée comme poche à urine à usage masculin, cette conduction ne pose normalement pas de difficulté particulière, dès lors que la verge de l'utilisateur est introduite à travers l'ouverture (dans le canal formé par le manchon) sur une longueur suffisante. De même, pour toutes les applications au recueillement de déchets, dans lesquelles les déchets sont introduits dans la poche à partir d'un organe ou d'embout saillant pouvant être introduit dans la poche à travers l'ouverture sur une longueur suffisante, la conduction des produits à l'intérieur de la poche ne pose pas de difficulté.

En revanche, des difficultés existent lorsque l'on souhaite utiliser les poches pour d'autres applications dans lesquelles l'organe à partir duquel les produits s'écoulent n'est pas du type pouvant être introduit sur une longueur suffisante dans le canal formé par le manchon. C'est par exemple le cas lorsque les poches sont utilisées comme poches à urine à usage féminin. En effet, dans ce cas, les produits, par exemple l'urine, sont introduits dans la poche à partir de l'extrémité du manchon qui fait saillie à l'extérieur de l'enveloppe. Ainsi, avant de parvenir à l'intérieur de l'enveloppe, ces produits s'écoulent contre la paroi du manchon. Il s'agit donc de faire en sorte que celle-ci procure une certaine étanchéité.

Une solution consisterait évidemment à réaliser le manchon à partir d'un tube à contour fermé qui, pour permettre le passage de la poche en configuration aplatie, serait aplati sur lui-même selon deux lignes de pliure parallèles à son axe longitudinal, délimitant les bords longitudinaux des deux parois du manchon aplati situé en vis-à-vis. Toutefois, ceci imposerait de fabriquer initialement le manchon en volume, puis de l'aplatir, ce qui grèverait les coûts de production. Par ailleurs, pour faire passer le manchon de sa configuration aplatie à sa configuration en canal, on presse les deux bords longitudinaux opposés du manchon aplati de manière à rapprocher ces bords l'un de l'autre. Pour que cette pression déforme les deux parois opposées en les écartant l'une de l'autre, il serait souhaitable que ces parois soient initialement légèrement convexes (vues de l'extérieur). Ceci pose des difficultés en termes de stockage des poches avant leur utilisation, puisque, si les parois du manchon aplati doivent rester quelques peu convexes, la poche n'est pas parfaitement aplatie et est donc plus encombrante qu'une poche réellement aplatie.

On pourrait également imaginer de réaliser le manchon à partir de deux éléments de paroi plans soudés l'un à l'autre selon leurs bords longitudinaux en vis-à-vis, pour avoir un contour fermé. Dans ce cas, cette soudure présente en elle-même une étape de fabrication qui augmente les coûts de production. De plus, partant de deux éléments plans soudés selon leurs bords longitudinaux, les chances qu'une pression sur les bords longitudinaux du manchon ainsi formé tende à conformer ce manchon en canal sont seulement de 50 %. En effet, une telle pression permet soit de rendre convexes les deux parois opposées, en réalisant ainsi le canal souhaité, soit de rendre convexe seulement l'une des parois, tandis que l'autre paroi adopte une forme concave et reste plaquée contre la paroi convexe. Dans ce dernier cas, le canal ne se forme pas et la manipulation d'une seule main est hasardeuse.

L'invention vise à remédier aux inconvénients précités en proposant une poche dont le manchon soit très simple à fabriquer et puisse être aisément manipulé de manière fiable, tout en permettant l'étanchéité relative souhaitée dans la partie de ce manchon qui, en configuration d'utilisation, forme un canal de conduction des déchets vers l'intérieur de la poche.

Ce but est atteint grâce au fait que la première paroi du manchon présente une première bande longitudinale d'extrémité en saillie latérale par rapport au bord de l'ouverture, tandis que la deuxième paroi présente un bord de glissement apte à glisser contre la face interne de ladite première bande longitudinale lors du passage de la poche de sa configuration aplatie à sa configuration d'utilisation.

Grâce à ces dispositions, lorsque l'on presse les bords longitudinaux du manchon de manière à les rapprocher l'un de l'autre, on déforme de manière certaine la première paroi du manchon en la rendant convexe, grâce au fait que la première bande longitudinale d'extrémité est en saillie latérale par rapport aux bords de l'ouverture. Cette première paroi étant rendue convexe, le bord de glissement de la deuxième paroi n'a pas d'autre choix que de glisser contre la face interne, qui est donc concave, de la première bande longitudinale. En conséquence, la deuxième paroi s'écarte de la première paroi, de sorte que ces deux parois forment entre elles le canal souhaité pour maintenir l'ouverture ouverte. De plus, le contact de glissement entre le bord de glissement et la deuxième paroi procure une certaine étanchéité évitant, lorsque les déchets sont introduits dans la poche, les fuites de ceux-ci par le bord du canal pourvu du bord de glissement et de la première bande longitudinale souhaitée.

La poche selon l'invention trouve de nombreuses applications. En effet, la facilité de manipulation du manchon pour le conformer en canal est utile pour tous les types d'utilisation. L'amélioration de la conduction des produits le long du canal dans la poche est en particulier utile lorsque les produits s'écoulent à partir d'organes ne pouvant pas être introduits dans la poche. Elle est également utile lorsque les produits s'écoulent à partir d'organes qui peuvent être introduits dans la poche, puisque l'invention permet d'éviter les fuites sans qu'il ne soit nécessaire d'apporter un soin particulier à la profondeur d'introduction des organes en question dans la poche.

Avantageusement, la deuxième paroi du manchon présente une deuxième bande longitudinale d'extrémité en saillie latérale par rapport au bord de l'ouverture, le bord de glissement étant formé sur cette deuxième bande.

Dans ce cas, la pression sur les bords longitudinaux opposés du manchon déforme également directement la deuxième paroi de manière à la rendre convexe, la deuxième bande longitudinale d'extrémité pouvant glisser par rapport à la première par le bord de glissement qui est formé sur cette deuxième bande et qui glisse contre la face interne de la première.

Il est cependant préférable que, sur au moins une portion, la première bande longitudinale dépasse davantage latéralement que la deuxième bande longitudinale par rapport au bord de l'ouverture.

Avantageusement, le bord de glissement est incliné, au moins dans un tronçon initial proche de l'ouverture, dans le sens allant en augmentant la largeur de la deuxième paroi en s'éloignant de l'ouverture. Ainsi, la base du bord de glissement adjacente à l'ouverture peut être pratiquement dans le prolongement du bord de l'ouverture, sans dépasser latéralement par rapport à ce bord, tandis que le bord de glissement s'écarte latéralement du bord de l'ouverture à mesure qu'il s'éloigne de l'ouverture dans le sens axial.

Lorsque l'on cherche à déformer le manchon, initialement aplati, pour conformer ses parois en canal, on exerce une pression sur les bords longitudinaux de ce manchon opposés, dans la région de la base de ce manchon, c'est-à-dire dans la région de l'ouverture de l'enveloppe. L'inclinaison du bord de glissement permet de faire en sorte que, par cette pression, le manchon forme une sorte de cornet, se déformant particulièrement aisément.

Avantageusement, la deuxième paroi présente un décrochement latéral vers l'extérieur, à la base du bord de glissement.

Ce décrochement latéral vers l'extérieur permet de caler la base du bord de glissement contre la base de la première bande longitudinale d'extrémité. On évite ainsi que, lors de la déformation du manchon, les deux parois de ce dernier ne se désaxent l'une par rapport à l'autre. Ceci favorise encore l'obtention de l'étanchéité relative souhaitée. De plus, ainsi qu'on le verra dans la suite, ce décrochement favorise le guidage des produits à l'intérieur de la poche.

Avantageusement, les deux parois sont fixées entre elles par leurs bords longitudinaux respectivement opposés à la première bande longitudinale et au bord de glissement.

En particulier, le manchon peut être fabriqué à partir d'un flan plat replié sur lui-même par une ligne de pliage qui forme un premier bord longitudinal des deux parois du manchon.

Cependant, on peut parfaitement prévoir que, sur les deux bords longitudinaux du manchon soient prévus la première bande longitudinale et le bord de glissement précités. Par exemple, la première paroi du manchon peut présenter une première bande longitudinale sur chacun de ses deux bords opposés, alors que la deuxième paroi présente un bord de glissement (et éventuellement une deuxième bande longitudinale) sur chacun de ses deux bords opposés. On peut également prévoir que la première paroi présente une première bande longitudinale sur un bord et un bord de glissement (éventuellement formé sur une deuxième bande longitudinale) sur l'autre bord et qu'il en soit de même pour l'autre paroi.

Avantageusement, la hauteur de dépassement du manchon par rapport à l'ouverture varie sur la largeur du manchon, entre une hauteur minimale mesurée au voisinage de l'une des extrémités latérales du manchon et une hauteur maximale mesurée au voisinage de l'extrémité latérale opposée.

Dans ce cas, avantageusement, la hauteur maximale est mesurée au voisinage de l'extrémité latérale du manchon à laquelle se trouvent la première bande longitudinale d'extrémité et le bord de glissement.

Avec la présente invention, une grande liberté de forme est laissée en ce qui concerne le bord libre du manchon opposé à l'ouverture. Ce bord peut être rectiligne, par exemple lorsque la poche est utilisée comme urinoir à usage masculin. Pour d'autres applications, par exemple lorsque la poche est utilisée comme un urinoir à usage féminin, une forme différente peut être donnée. Par exemple, en faisant varier la hauteur de dépassement du manchon, on permet une adaptation du bord libre de ce dernier à l'anatomie féminine. En effet, cette variation de hauteur peut être choisie de telle sorte que, lorsque le manchon est conformé en canal, le bord libre de ce dernier puisse se plaquer contre la vulve de la femme qui utilise la poche.

Avantageusement, la poche comprend des moyens pour maintenir les parois du manchon dans leur conformation formant un canal.

Par exemple, le manchon comporte deux languettes de maintien, respectivement portées par la première et par la deuxième paroi du manchon du côté opposé à l'ouverture de la poche, chacune de ces languettes présentant une tête libre séparée de la paroi qui la porte par une fente de blocage.

Par cette simple conformation, en conformant les parois du manchon en canal, on peut amener la languette portée par la première paroi du manchon à l'intérieur de la languette portée par la deuxième paroi, alors que la première bande longitudinale qui est portée par la première paroi est, à l'inverse, située du côté extérieur de la deuxième paroi. Les deux parois sont alors maintenues l'une par rapport à l'autre.

Avantageusement, le manchon présente un tronçon interne à l'enveloppe, ayant des pattes d'écartement, respectivement formées dans la continuité de chacune des deux parois du manchon et ayant des extrémités latérales libres aptes à s'écarter l'une de l'autre dans la configuration d'utilisation de la poche pour espacer les faces opposées de l'enveloppe souple.

De telles pattes d'écartement sont connues par la demande de brevet européen n° 0 847 742. Elles s'avèrent être compatibles avec la présente invention, permettant ainsi la réalisation d'une poche qui combine les différents avantages précités, avec un écartement sûr des feuilles qui constituent l'enveloppe, même si ces feuilles sont très souples, voire initialement légèrement collées entre elles.

Il est encore avantageux que, dans une région opposée à l'ouverture, l'enveloppe présente une ligne d'affaiblissement pouvant être rompue pour former une déchirure s'entendant jusqu'à l'espace interne de l'enveloppe.

Ainsi, la poche peut être vidée par une manipulation simple.

L'invention sera bien comprise et ses avantages apparaîtront mieux à la lecture de la description détaillée qui suit, d'un mode de réalisation représenté à titre d'exemple non limitatif. La description se réfère aux dessins annexés sur lesquels :
- la figure 1 est une vue en élévation d'une poche conforme à l'invention, dans sa configuration aplatie ;
- la figure 2 est une vue en plan du flan dans lequel est réalisé le manchon de la poche de la figure 1;
- la figure 3 est une vue en perspective montrant la forme du manchon en configuration d'utilisation de la poche de la figure 1 ;
- la figure 4 est une vue de la portion d'une poche proche de son ouverture, selon une variante de réalisation, particulièrement adaptée à l'usage comme urinal féminin ;
- la figure 5 est une vue en plan du flan dans lequel est réalisé le manchon de la poche de la figure 1 ;
- la figure 6 est une vue en perspective montrant la forme du manchon en configuration d'utilisation de la poche de la figure 4 ;
- la figure 7 est une vue en plan du flan dans lequel est réalisé un manchon selon une variante de réalisation ; et
- la figure 8 est une vue en perspective qui montre le manchon selon la variante de la figure 7, alors que ce manchon est conformé en canal.

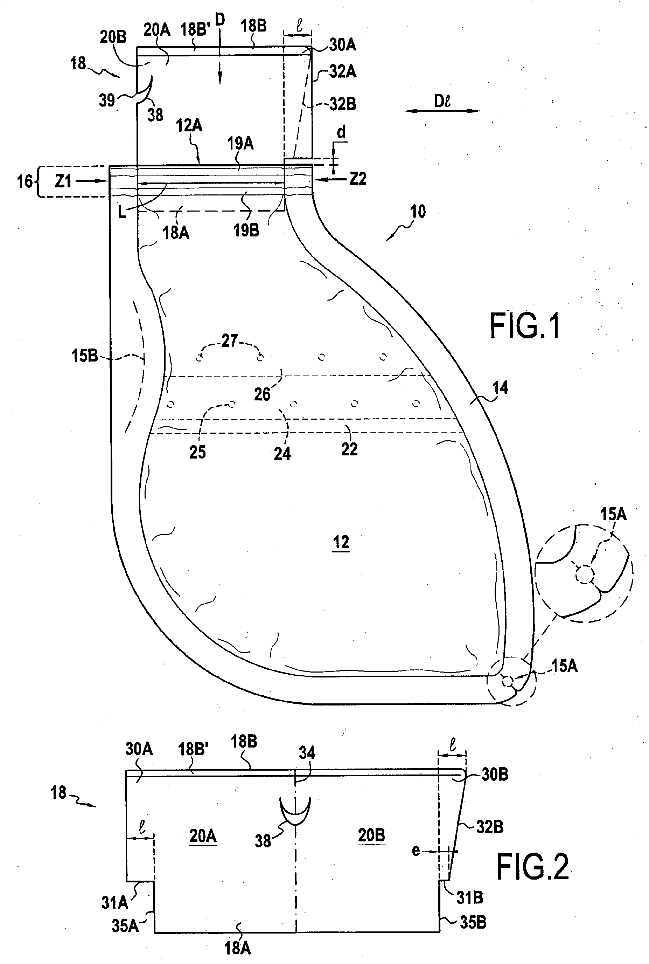

La poche 10 représentée sur la figure 1 comprend une enveloppe souple 12. En particulier, cette enveloppe est réalisée à partir de deux feuilles minces de matière plastique découpées de manière convenable et soudées l'une à l'autre sur la quasi-totalité du contour, par une ligne de soudure 14. L'enveloppe 12 présente une ouverture 12A ménagée par une interruption de la ligne de soudure 14, pour permettre à l'espace intérieur de l'enveloppe de communiquer avec l'extérieur.

Dans l'exemple représenté, cette ouverture est ménagée à l'extrémité libre d'une portion de col 16 que présente l'enveloppe, dans laquelle la largeur de cette enveloppe est réduite.

La poche comprend un manchon de renfort 18 qui est fixé en travers de l'ouverture 12A et fait saillie à l'extérieur de l'enveloppe.

En l'espèce, le manchon comprend non seulement sa partie principale de manchon proprement dit, qui fait saillie au-delà de l'ouverture 12, mais également une portion interne 18A, qui est insérée dans l'ouverture de manière à s'étendre sur toute la largeur L de cette dernière.

Par exemple, les feuilles qui forment l'enveloppe sont en matière plastique souple, tandis que le manchon est en matière plastique plus rigide. Ainsi, pour opérer la fixation du manchon avec l'enveloppe, il suffit de souder ou coller les feuilles de l'enveloppe aux parois du manchon. On voit ainsi sur la figure 1 deux lignes de soudure, respectivement 19A et 19B orientées transversalement à la direction D d'introduction des déchets dans la poche, lignes sur lesquelles la paroi avant 20A du manchon, visible sur la figure 1, est soudée à la feuille correspondante de l'enveloppe. Bien entendu, la paroi arrière 20B du manchon est soudée de la même manière à la feuille opposée de la poche.

On constate également sur la figure 1 que, dans une région opposée à l'ouverture, la ligne de soudure 14 présente une ligne d'affaiblissement 15A, réalisée par exemple à l'aide de micro perçages en pointillées. Cet affaiblissement s'étend seulement sur la ligne de soudure, sans atteindre l'espace interne de l'enveloppe. Lorsque la poche est pleine de déchets, ceux-ci peuvent ainsi être vidés en déchirant l'enveloppe selon la ligne d'affaiblissement 15A, avec un effort suffisant pour que la déchirure ainsi créée s'étende jusqu'à l'espace interne de l'enveloppe.

Par ailleurs, sur l'un de ces côtés voisins de l'ouverture, la ligne de soudure 14 présente une autre ligne d'affaiblissement 15B, pouvant être également réalisée par des perçages discontinus. Cette ligne est à distance de l'espace interne de l'enveloppe et elle est orientée de telle sorte que sa déchirure ne peut pas se propager jusqu'à l'intérieur de l'enveloppe. Ainsi, lorsque la poche est pleine et que l'on souhaite la stocker avant de la vider, la ligne 15B peut être déchirée pour ménager une fente permettant d'accrocher la poche à un crochet ou analogue.

On voit encore sur la figure 1 que la poche présente un clapet de sécurité du type décrit dans la demande de brevet européen n° 0 847 742. Ainsi, ce clapet est constitué de plusieurs feuilles internes disposées contre chacune des parois de la poche. Dans l'exemple représenté, le clapet comporte, pour chacune des deux parois de la poche, une première paire de feuilles 22 qui s'étend à partir de l'ouverture 12A jusqu'à une région médiane de la poche, une deuxième paire de feuilles 24 qui s'étend à l'intérieur des feuilles de la première paire 22, et une troisième paire de feuilles 26 qui s'étend à l'intérieur de la paire de feuilles 24, les feuilles 26 étant plus courtes que les feuilles 24, elles-mêmes plus courtes que les feuilles 22. Ces feuilles s'étendent sur toute la largeur de la poche dans la région où elles se trouvent, et sont donc soudées entre elles et avec les parois de l'enveloppe, par la ligne de soudure 14. Par ailleurs, les feuilles de la paire 24 sont soudées entre elles par des points de soudure 25, et les feuilles des paires 26 et 24 sont soudées par des points de soudure 27 décalés par rapport aux points de soudure 25. Ces différentes paires de feuilles astucieusement soudées constituent un clapet anti-reflux, qui empêche le reflux de déchets cités dans la poche lorsque celle-ci est manipulée après son utilisation.

On constate que la ligne d'affaiblissement 15B se trouve dans une région de la ligne de soudure dans laquelle toutes les différentes couches de feuilles sont présentes, de sorte que cette soudure est particulièrement résistante, ceci convenant à l'accrochage précédemment évoqué.

L'invention s'intéresse tout particulièrement au manchon 18. Comme indiqué précédemment, il est soudé en travers de l'ouverture, sa partie interne 18A s'étendant sur toute la largeur L de l'ouverture 12A. On voit sur la figure 1 que la paroi avant 20A du manchon, ci-après dénommée "première paroi" présente une première bande longitudinale d'extrémité 30A qui est en saillie latérale par rapport au bord de l'ouverture 12A. Au sens de la présente demande de brevet, la direction longitudinale est la direction D d'introduction des déchets dans la poche, elle-même définie par l'axe du manchon. La direction latérale Dl est donc perpendiculaire à cette direction longitudinale. On voit sur la figure 1 que la première bande longitudinale 30A dépasse latéralement d'une largeur l par rapport au bord de l'ouverture. En se reportant à la figure 2, on comprend que la partie interne 18A du manchon est délimitée par rapport à la partie de ce manchon qui dépasse en dehors de l'ouverture lorsque le manchon est en place dans la poche, par un décrochement 31A de largeur l.

De son côté, la deuxième paroi 20B du manchon présente un bord de glissement 32B qui est apte à glisser contre la face interne de la première bande 30A lors du passage de la poche de sa configuration aplatie à sa configuration d'utilisation.

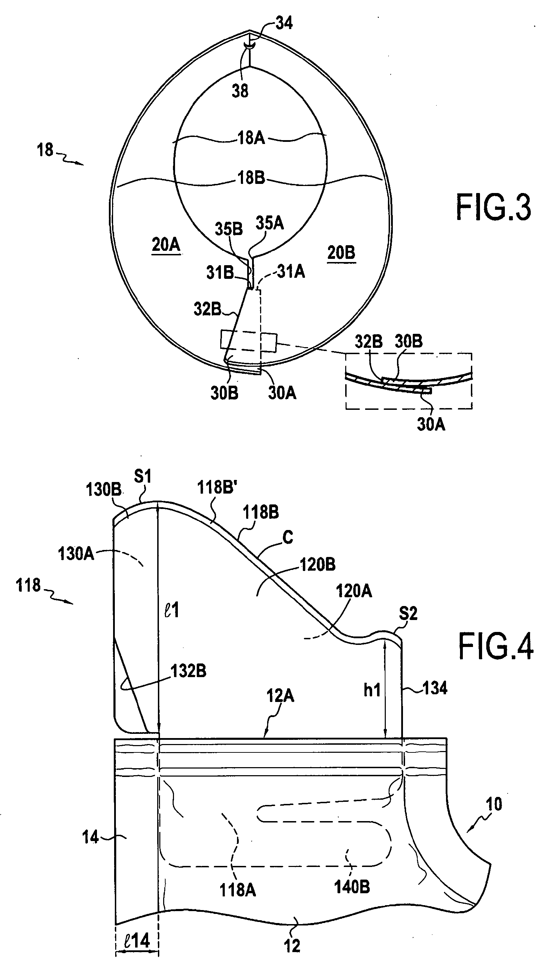

La figure 3 permet de bien comprendre ce phénomène, car elle montre la forme adoptée par le manchon lors de sa mise en forme de canal pour provoquer le passage de la poche de sa configuration aplatie à sa configuration d'utilisation. Pour provoquer ce changement de forme du manchon, l'utilisateur exerce une pression dans les zones Z1 et Z2 indiquées sur la figure 1, situées à la base du manchon. Sous l'effet de cette pression qui tend à rapprocher l'un de l'autre les bords longitudinaux opposés du manchon, les parois du manchon se courbent, la première paroi 20A adoptant une forme convexe tandis que sa bande longitudinale d'extrémité, qui forme une extension libre de cette première paroi, adopte également une forme convexe mais avec une courbure moindre. Sous l'effet de la pression exercée dans les zones Z1 et Z2, la face intérieure de la première paroi 20A exerce un effort de réaction contre le bord libre 32B et, du fait de la forme particulière adoptée par la première bande longitudinale 30A, le bord de glissement n'a d'autre choix que de glisser sur cette face interne, pour conformer la deuxième paroi 20B selon une forme convexe. Ainsi, les deux parois délimitent entre elles le canal provoquant le maintien de l'ouverture de la poche en situation ouverte.

En l'espèce, le bord de glissement 32B est formé sur le bord d'une deuxième bande longitudinale d'extrémité 30B qui fait partie de la deuxième paroi du manchon et est en saillie latérale par rapport au bord de l'ouverture. Sur au moins un tronçon, la bande 30B dépasse moins latéralement que la bande 30A. En l'espèce, le dépassement latéral de la bande 30B varie du fait de l'inclinaison du bord de glissement 32B. La valeur maximale de ce dépassement latéral correspond en l'espèce à la largeur l de la première bande 30A, cette largeur étant constante en l'espèce.

On voit en particulier sur la figure 2 que la deuxième paroi 20B présente un décrochement latéral vers l'extérieur 31B qui est situé à la base du bord de glissement 32B, c'est-à-dire dans la région de ce bord de glissement la plus proche de l'ouverture 12A de la poche. Ce décrochement 31B peut avoir une largeur e très faible, par exemple de l'ordre de 5 à 20 %, de préférence de l'ordre de 10 %, de la largeur l. Ainsi, les largeurs e et l peuvent respectivement être de l'ordre de 1 à 2 mm et de 8 à 15mm pour des poches servant d'urinal à usage humain.

Comme on le voit sur les figures 2 et 3, les deux parois 20A et 20B sont fixées entre elles par leurs bords longitudinaux respectivement opposés à la première bande longitudinale 30A et au bord de glissement 32B. En fait, dans l'exemple représenté, le manchon 18 est réalisé à partir d'un flan en matière plastique qui plié est selon une ligne de pliage 34 pour mettre le manchon à plat comme dans la situation de la figure 1. Cette ligne de pliage 34 forme les bords longitudinaux communs des parois 20A et 20B qui sont liés entre eux. On pourrait également prévoir de réaliser le manchon à partir de deux pièces plates, c'est-à-dire sous la forme de deux flans distincts, soudés entre eux selon leur ligne commune pour former la ligne de pliage 34.

Ainsi, avec l'invention, les bords du manchon opposés à la ligne 34, c'est-à-dire les bords qui sont pourvus de la bande 30A et du bord de glissement 30B sont libres de se déplacer l'un par rapport à l'autre. Ceci concerne toute la partie du manchon qui fait saillie au-delà de l'ouverture 12A. En effet, pour ce qui concerne la partie interne 18A du manchon, on a indiqué précédemment que la largeur L de cette partie interne en configuration aplatie correspond à celle de l'ouverture 12A. Ainsi, pour cette partie interne, les bords 35A et 35B sont en fait incapables ou pratiquement incapables de se déplacer l'un par rapport à l'autre, du fait de la présence de la ligne de soudure 14. Sur les côtés de l'ouverture 12A, cette ligne 14 peut d'ailleurs légèrement empiéter sur le manchon c'est-à-dire sur la partie de la ligne de pliage 34 qui s'étend sur la partie interne 18A du manchon et sur les bords 35A et 35B précédemment évoqués.

Dans l'exemple représenté sur les figures 1 à 3, la hauteur de dépassement du manchon par rapport à l'ouverture est sensiblement constante. Bien entendu, ceci n'empêche pas que le bord supérieur 18B du manchon 18 puisse légèrement être adouci si nécessaire. Ainsi, il peut être chanfreiné ou bien pourvu d'un bourrelet 18B'.

En référence aux figures 4 à 6, on décrit maintenant une variante de réalisation du manchon. Sur la figure 4, on a seulement représenté la partie de la poche proche de son ouverture 12A. Le manchon 118 est analogue au manchon 18 précédemment décrit mais il s'en distingue en deux aspects. D'une part, le bord libre 118B du manchon 118 opposé à l'ouverture 12A a une conformation particulière, en particulier adaptée à son utilisation comme urinale pour les femmes. On relève d'ores et déjà que ce bord libre peut présenter un bourrelet ou analogue 118B' le rendant agréable au toucher. D'autre part, la partie interne 118A du manchon présente des pattes d'écartement qui seront décrites ci-après.

Les différents éléments constitutifs du manchon 118 sont désignés par les mêmes références que pour le manchon 18, augmentés de 100. En se référant aux figures 4 à 6, on voit donc que le manchon 118 présente une première paroi 120A et une deuxième paroi 120B, reliées par une ligne de pliage 134. La première paroi 120A présente une première bande longitudinale d'extrémité 130A, tandis que la deuxième paroi 120B présente un bord de glissement 132B ménagé sur le bord d'une deuxième bande longitudinale 130B. On voit sur la figure 4 que les deux bandes longitudinales dépassent latéralement de l'ouverture, c'est-à-dire qu'elles sont situées en saillie latérale par rapport au bord latéral de l'ouverture délimité par la ligne de soudure 14. La première bande longitudinale 130A se situe globalement dans le prolongement de la ligne de soudure 14 et présente une largeur correspondant approximativement à celle de cette ligne de soudure. La deuxième bande longitudinale 130B présente une portion terminale libre 130B' de largeur sensiblement constante, correspondant à la largeur 114 de la ligne de soudure 14. Entre le bord de l'ouverture 12A et cette portion terminale 130B', cette deuxième bande longitudinale d'extrémité présente le bord de glissement 132B qui, comme le bord de glissement 32B de la précédente variante, est incliné pour mieux glisser contre la face interne de la première bande longitudinale 130A. Des décrochements 131A et 131B, respectivement analogues aux décrochements 31A et 31B précédemment évoqués, sont situés à la base des bandes longitudinales 130A et 130B.

Comme pour la variante des figures précédentes, celle des figures 4 à 6 prévoit que le manchon soit réalisé à partir d'un flan plié. Les deux parois 120A et 120B sont donc reliées par la, ligne de pliage 134. On voit sur la figure 4 que la hauteur de dépassement du manchon 118 par rapport à l'ouverture 12A varie sur la largeur du manchon, entre une hauteur minimale h1 mesurée au voisinage de l'une des extrémités latérales du manchon, et une hauteur maximale h2 mesurée au voisinage de l'autre extrémité latérale. En l'espèce, la hauteur maximale h2 est mesurée au voisinage de l'extrémité latérale du manchon à laquelle se trouvent la première bande longitudinale d'extrémité 130A et le bord de glissement 132B.

Comme on le voit sur la figure 4, en configuration aplatie de la poche, le bord libre 118B du manchon opposé à l'ouverture 12A présente une forme sensiblement en S avec une portion centrale C en forme de rampe montante, de deux portions terminales respectivement S1 et S2 qui sont légèrement arrondies. Cette forme est particulièrement adaptée à l'usage de la poche comme urinal pour les femmes. Pour utiliser la poche, il suffit de déformer le manchon pour le conformer en canal, et de placer ce manchon de telle sorte que la partie de plus petite hauteur, correspondant à la portion S2, se trouve contre la partie postérieure de la vulve.

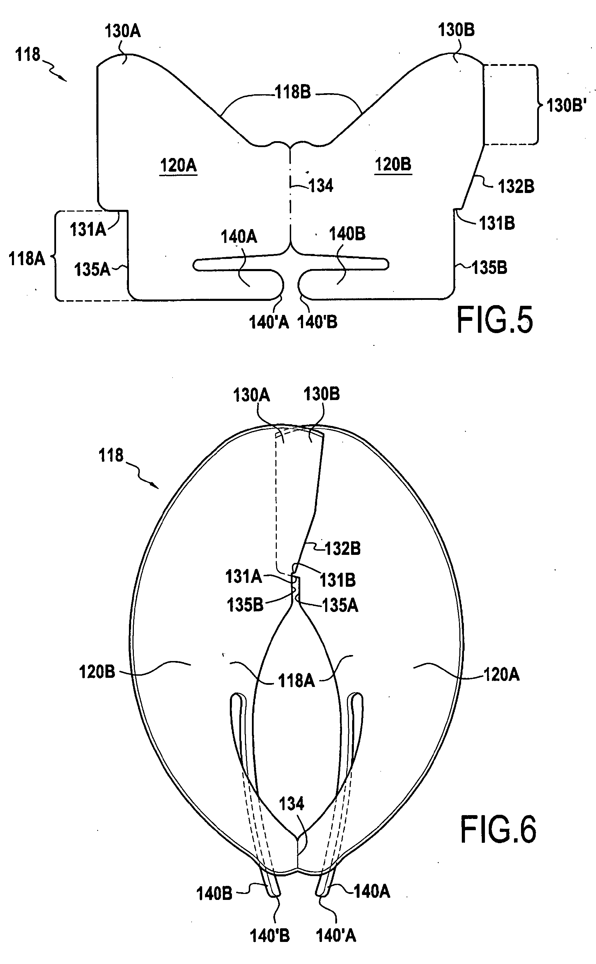

Dans la variante des figures 4 à 6, le manchon présente en outre des pattes d'écartement. Plus précisément, la partie 118A du manchon qui forme un tronçon de ce dernier interne à l'enveloppe 12, présente des pattes d'écartement 140A et 140B. Ces pattes sont respectivement formées dans la continuité de chacune des deux parois du manchon et ont des extrémités latérales libres, respectivement 140'A et 140'B qui sont aptes à s'écarter l'une de l'autre dans la configuration d'utilisation de la poche pour espacer les faces opposées de l'enveloppe souple, comme décrit dans la demande de brevet européen n° 0 847 742 et comme on le comprend en considérant la figure 6.

La poche selon l'invention comprend avantageusement des moyens pour maintenir les parois du manchon dans leur conformation formant un canal. A cet effet, il est par exemple possible de pratiquer dans le manchon une découpe du type de la découpe 38 représentée sur la figure 2. Cette découpe est symétrique par rapport à la ligne de pliage et présente la forme d'un sourire. Ainsi, au-dessus de la découpe, est ménagée une languette 39 qui peut être repliée vers l'intérieur ou vers l'extérieur du manchon pour former une cassure dans la ligne de pliage s'opposant à l'aplatissement du manchon.

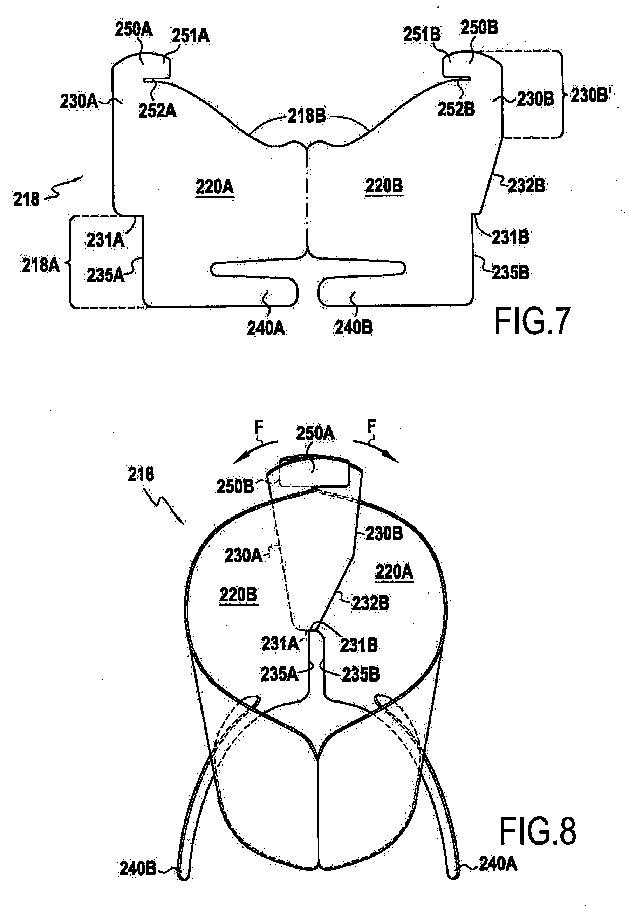

En référence aux figures 7 et 8, on décrit maintenant une variante de réalisation du manchon, comprenant des moyens pour maintenir les parois du manchon dans leur conformation formant un canal, selon une autre réalisation.

Sur les figures 7 et 8, on a utilisé les mêmes références que sur les figures 4 à 6, augmentées de 100. Le manchon 218 représenté sur les figures 7 et 8 est analogue au manchon 118, et l'on reconnaît notamment les deux parois 220A et 220B, les bandes longitudinales 230A et 230B, le bord de glissement 232B, les décrochements latéraux 231A et 231B, et les pattes d'écartement 240A et 240B.

Cependant, le manchon 218 se distingue du manchon 118 en ce qu'il comporte deux languettes de maintien 250A et 250B, respectivement portées par la première paroi 220A et par la deuxième paroi 220B. Chacune de ces languettes présente une tête libre, respectivement 251A et 251B, séparée de la paroi qui la porte, respectivement 220A et 220B, par une fente, respectivement 252A et 252B. Ces fentes sont globalement orientées sensiblement transversalement et sont orientées de telle sorte que la tête libre 251A de la languette 250A est tournée du côté allant en s'éloignant de la première bande longitudinale 230A, tandis que la tête libre 251B de la languette 250B est tournée du côté allant en s'éloignant du bord de glissement 232B.

En l'espèce, la partie de rattachement de la languette 250A à la première paroi 220A est sensiblement dans le prolongement longitudinal de la première bande longitudinale 230A et la partie de rattachement de la languette 250B à la deuxième paroi 220B est sensiblement dans le prolongement longitudinal de la deuxième bande longitudinale 230B.

Comme on le voit sur la figure 8, lorsque lé manchon est conformé en canal, les fentes 252A et 252B peuvent être engagées l'une dans l'autre. Comme on l'a indiqué, pour conformer le manchon en canal, le bord de glissement 232B a glissé contre la face interne de la première bande longitudinale 230A. Donc, comme dans tous les modes de réalisation la partie de la deuxième paroi adjacente au bord de glissement (cette partie étant la deuxième bande longitudinale 30B, 130B ou 230B lorsqu'elle est présente), est située du côté interne de la première paroi du manchon, lorsque ce dernier est dans sa conformation en canal.

Grâce à l'interpénétration des fentes 252A et 252B, cette situation est inversée au niveau des languettes 250A et 250B. En effet, c'est la première languette 250A, portée par la première paroi 220A, qui se retrouve du côté intérieur de la languette 250B, portée par la deuxième paroi 220B. De ce fait, chacune des languettes est retenue par la fente de l'autre languette. Ainsi, les languettes sont empêchées de s'éloigner l'une de l'autre dans le sens indiqué par les flèches F sur la figure 8, ce qui fait que le manchon est contraint de rester dans sa conformation en canal.

Faire ainsi coopérer les languettes 250A et 250B est très simple. Il suffit, après avoir conformé le manchon en canal, de les saisir entre deux doigts pour les faire glisser l'une contre l'autre jusqu'à faire passer la languette 250B derrière la languette 250A.

On relève par ailleurs en référence à la figure 8, que les languettes superposées offrent une surface de prise qui peut être pincée par l'utilisateur pour maintenir le manchon en position adéquate pendant l'utilisation de la poche, en particulier pendant la miction si la poche est utilisée comme urinal.

Bien entendu, les languettes de maintien sont parfaitement compatibles avec la variante de réalisation des figures 1 à 3, en lieu et place de la découpe 38 qui a été décrite.

Quelle que soit la variante retenue, le manchon de la poche selon l'invention présente la particularité de passer très facilement de sa configuration aplatie à sa configuration en canal. De plus, bien que le bord de glissement soit libre de glisser par rapport à la première bande longitudinale, de sorte que le manchon n'est pas soudé dans cette zone, cette coopération de glissement procure une étanchéité. D'une part, le bord de glissement est en contact avec la face interne de la première paroi sur toute sa longueur et évite donc les fuites intempestives sur toute cette zone de contact. Par ailleurs, le décrochement 31B ou 131B qui est situé à la base du bord de glissement a pour effet de guider le liquide ruisselant contre ce bord vers la face interne de la paroi 20B et donc vers l'intérieur de la poche. Il faut noter qu'on a quelque peu exagéré sur les dessins la distance d entre le bord supérieur de l'ouverture et la base de la première bande longitudinale ou du bord de glissement (c'est-à-dire la base du décrochement 31A, 31B ou 131A, 131B). En l'espèce, la ligne de soudure 119A est en réalité réalisée de manière à parvenir pratiquement au niveau de ces bases. De cette manière, lorsque le liquide parvient à la base du bord de glissement 32B, il est pratiquement dans l'enveloppe.

De même, lorsque le manchon est conformé en canal, les bords longitudinaux de la partie interne de ce manchon (35A et 35B ou 135A et 135B) se touchent, ce qui contribue à l'effet d'étanchéité. Ces bords sont représentés espacés sur les figures 3 et 5, pour la clarté du dessin.

## Revendications

1. Poche (10) comprenant une enveloppe souple (12) ayant une ouverture (12A) dont le bord est fixé aux deux parois opposées d'un manchon de renfort (18 ; 118) en saillie à l'extérieur de l'enveloppe, la poche étant susceptible d'adopter une configuration aplatie dans laquelle lesdites parois sont situées l'une contre l'autre, et une configuration d'utilisation dans laquelle ces parois forment un canal qui maintient l'ouverture ouverte,
**caractérisée en ce que** la première paroi (20A ; 120A) du manchon (18 ; 118) présente une première bande longitudinale d'extrémité (30A ; 130A) en saillie latérale par rapport au bord de l'ouverture, tandis que la deuxième paroi (20B ; 120B) présente un bord de glissement (32B ; 132B) apte à glisser contre la face interne de ladite première bande longitudinale lors du passage de la poche de sa configuration aplatie à sa configuration d'utilisation.

2. Poche selon la revendication 1, **caractérisée en ce que** la deuxième paroi (20B ; 120B) du manchon (18 ; 118) présente une deuxième bande longitudinale d'extrémité (30B ; 130B) en saillie latérale par rapport au bord de l'ouverture (12A), le bord de glissement (32B ; 132B) étant formé sur cette deuxième bande.

3. Poche selon la revendication 2, **caractérisée en ce que** le bord de glissement (32B ; 132B) est incliné, au moins dans un tronçon initial proche de l'ouverture (12A), dans le sens allant en augmentant la largeur de la deuxième paroi en s'éloignant de l'ouverture.

4. Poche selon l'une quelconque des revendications 1 à 3, **caractérisée en ce que** la deuxième paroi (20B ; 120B) présente un décrochement latéral (31B ; 131B) vers l'extérieur, à la base du bord de glissement (32B ; 132B).

5. Poche selon l'une quelconque des revendications 1 à 4, **caractérisée en ce que** les deux parois (20A, 20B ; 120A, 120B) sont fixées entre elles par leurs bords longitudinaux respectivement opposés à la première bande longitudinale (30A ; 130A) et au bord de glissement (32B ; 132B).

6. Poche selon l'une quelconque des revendications 1 à 5, **caractérisée en ce que** la hauteur de dépassement du manchon (118) par rapport à l'ouverture (12A) varie sur la largeur du manchon, entre une hauteur minimale (h1) mesurée au voisinage de l'une des extrémités latérales du manchon et une hauteur maximale (h2) mesurée au voisinage de l'extrémité latérale opposée.

7. Poche selon la revendication 6, **caractérisée en ce que** la hauteur maximale (h2) est mesurée au voisinage de l'extrémité latérale du manchon (118) à laquelle se trouvent la première bande longitudinale d'extrémité (130A) et le bord de glissement (132B).

8. Poche selon la revendication 6 ou 7, **caractérisée en ce que**, en configuration aplatie de la poche, le bord libre (118B) du manchon (118) opposé à l'ouverture (12A), présente une forme en S.

9. Poche selon l'une quelconque des revendications 1 à 8, **caractérisée en ce qu'**elle comprend des moyens (38, 39) pour maintenir les parois du manchon dans leur conformation formant un canal.

10. Poche selon la revendication 9, **caractérisée en ce que** le manchon (218) comporte deux languettes de maintien (250A, 250B), respectivement portées par la première et par la deuxième paroi (220A, 220B) du manchon du côté opposé à l'ouverture de la poche, chacune de ces languettes (250A, 250B) présentant une tête libre (251A, 251B) séparée de la paroi qui la porte par une fente de blocage (252A, 252B).

11. Poche selon l'une quelconque des revendications 1 à 10, **caractérisée en ce que** le manchon (118) présente un tronçon (118A) interne à l'enveloppe, ayant des pattes d'écartement (140A, 140B), respectivement formées dans la continuité de chacune des deux parois du manchon et ayant des extrémités latérales libres (140'A, 140'B) aptes à s'écarter l'une de l'autre dans la configuration d'utilisation de la poche pour espacer les faces opposées de l'enveloppe souple (12).

12. Poche selon l'une quelconque des revendications 1 à 11, **caractérisée en ce que**, dans une région opposée à l'ouverture (12A), l'enveloppe (12) présente une ligne d'affaiblissement (15A) pouvant être rompue pour former une déchirure s'entendant jusqu'à l'espace interne de l'enveloppe.

## Patentansprüche

1. Tasche (10), umfassend eine flexible Hülle (12), die eine Öffnung (12A) aufweist, deren Rand an den zwei gegenüberliegenden Wänden einer Verstärkungsmuffe (18; 118) befestigt ist, die außerhalb der Hülle vorsteht, wobei die Tasche geeignet ist, eine abgeflachte Konfiguration, in der die Wände aneinander angeordnet sind, und eine Verwendungskonfiguration anzunehmen, in der diese Wände einen Kanal bilden, der die Öffnung offen hält,
**dadurch gekennzeichnet, dass** die erste Wand (20A; 120A) der Muffe (18; 118) einen ersten länglichen Endstreifen (30A; 130A) aufweist, der seitlich gegenüber dem Rand der Öffnung vorsteht, während die zweite Wand (20B; 120B) einen Gleitrand (32B; 132B) aufweist, der geeignet ist, an der Innenfläche des ersten länglichen Streifens während des Übergangs der Tasche von ihrer abgeflachten Konfiguration in ihre Verwendungskonfiguration zu gleiten.

2. Tasche nach Anspruch 1, **dadurch gekennzeichnet, dass** die zweite Wand (20B; 120B) der Muffe (18; 118) einen zweiten länglichen Endstreifen (30B; 130B) aufweist, der seitlich gegenüber dem Rand der Öffnung (12A) vorsteht, wobei der Gleitrand (32B; 132B) auf diesem zweiten Streifen gebildet ist.

3. Tasche nach Anspruch 2, **dadurch gekennzeichnet, dass** der Gleitrand (32B; 132B) mindestens in einem Anfangsabschnitt in der Nähe der Öffnung (12A) in der Richtung geneigt ist, die durch Vergrößern der Breite der zweiten Wand von der Öffnung weg verläuft.

4. Tasche nach einem der Ansprüche 1 bis 3, **dadurch gekennzeichnet, dass** die zweite Wand (20B; 120B) einen seitlichen Versatz (31 B; 131 B) zur Außenseite an der Basis des Gleitrandes (32B; 132B) aufweist.

5. Tasche nach einem der Ansprüche 1 bis 4, **dadurch gekennzeichnet, dass** die zwei Wände (20A, 20B; 120A, 120B) untereinander durch ihre jeweiligen Längsränder befestigt sind, die dem ersten länglichen Streifen (30A; 130A) und dem Gleitrand (32B; 132B) gegenüber liegen.

6. Tasche nach einem der Ansprüche 1 bis 5, **dadurch gekennzeichnet, dass** die Überstandshöhe der Muffe (118) gegenüber der Öffnung (12A) auf der Breite der Muffe zwischen einer minimalen Höhe (h1), die in der Nähe von einem der seitlichen Enden der Muffe gemessen wird, und einer maximalen Höhe (h2), die in der Nähe des gegenüberliegenden seitlichen Endes gemessen wird, variiert.

7. Tasche nach Anspruch 6, **dadurch gekennzeichnet, dass** die maximale Höhe (h2) in der Nähe des seitlichen Endes der Muffe (118) gemessen wird, an der sich der erste längliche Endstreifen (130A) und der Gleitrand (132B) befinden.

8. Tasche nach Anspruch 6 oder 7, **dadurch gekennzeichnet, dass** in der abgeflachten Konfiguration der Tasche der freie Rand (118B) der Muffe (118), der der Öffnung (12A) gegenüberliegt, eine S-Form aufweist.

9. Tasche nach einem der Ansprüche 1 bis 8, **dadurch gekennzeichnet, dass** sie Mittel (38, 39) aufweist, um die Wände der Muffe in ihrer Konfiguration zu halten, die einen Kanal bildet.

10. Tasche nach Anspruch 9, **dadurch gekennzeichnet, dass** die Muffe (218) zwei Haltezungen (250A, 250B) aufweist, die jeweils von der ersten und von der zweiten Wand (220A, 220B) der Muffe auf der Seite, die der Öffnung der Tasche gegenüberliegt, getragen werden, wobei jede dieser Zungen (250A, 250B) einen freien Kopf (251 A, 251 B) aufweist, der von der Wand, die sie trägt, durch einen Blockierschlitz (252A, 252B) getrennt ist.

11. Tasche nach einem der Ansprüche 1 bis 10, **dadurch gekennzeichnet, dass** die Muffe (118) einen Abschnitt (118A) im Inneren der Hülle aufweist, der Spreizlaschen (140A, 140B) aufweist, die jeweils in der Kontinuität von jeder der zwei Wände der Muffe gebildet sind und freie seitliche Enden (140'A, 140'B) aufweisen, die geeignet sind, sich in der Verwendungskonfiguration der Tasche voneinander weg zu spreizen, um die gegenüberliegenden Flächen der flexiblen Hülle (12) zu beabstanden.

12. Tasche nach einem der Ansprüche 1 bis 11, **dadurch gekennzeichnet, dass** die Hülle (12) in einem Bereich, der der Öffnung (12A) gegenüberliegt, eine Schwächungslinie (15A) aufweist, die gebrochen werden kann, um einen Riss zu bilden, der sich bis zu dem Innenraum der Hülle erstreckt.

## Claims

1. A pouch (10) comprising a flexible bag (12) having an opening (12A) with its edge fastened to two opposite walls of a reinforcing sleeve (18; 118) projecting to the outside of the bag, the pouch being suitable for adopting a flat configuration in which said walls are situated one against the other, and a utilization configuration in which the walls form a channel that holds the opening open, the pouch being **characterized in that** the first wall (20A; 120A) of the sleeve (18; 118) presents a first longitudinal end strip (30A; 130A) projecting laterally from the edge of the opening, while the second wall (20B; 120B) presents a sliding edge (32B; 132B) suitable for sliding against the inside face of said first longitudinal strip when the pouch is passing from its flat configuration to its utilization configuration.

2. A pouch according to claim 1, **characterized in that** the second wall (20B; 120B) of the sleeve (18; 118) presents a second longitudinal end strip (30B; 130B) projecting laterally from the edge of the opening (12A), the sliding edge (32B; 132B) being formed on the second strip.

3. A pouch according to claim 2, **characterized in that** the sliding edge (32B; 132B) is inclined, at least in an initial segment close to the opening (12A), in the direction that increases the width of the second wall on going away from the opening.

4. A pouch according to any one of claims 1 to 3, **characterized in that** the second wall (20B; 120B) presents an outwardly-directed lateral step (31B; 131B) at the base of the sliding edge (32B; 132B).

5. A pouch according to any one of claims 1 to 4, **characterized in that** the two walls (20A, 20B; 120A, 120B) are fastened together via their longitudinal edges respectively opposite from the first longitudinal strip (30A; 130A) and from the sliding edge (32B; 132B).

6. A pouch according to any one of claims 1 to 5, **characterized in that** the height by which the sleeve (118) projects beyond the opening (12A) varies across the width of the sleeve between a minimum height (h1) measured in the vicinity of one of the lateral ends of the sleeve and a maximum height (h2) measured in the vicinity of the opposite lateral end.

7. A pouch according to claim 6, **characterized in that** the maximum height (h2) is measured in the vicinity of the lateral end of the sleeve (118) where the first longitudinal end strip (130A) and the sliding edge (132B) are located.

8. A pouch according to claim 6 or claim 7, **characterized in that** when the pouch is in the flat configuration, the free edge (118B) of the sleeve (118) opposite from the opening (12A) represents an S-shape.

9. A pouch according to any one of claims 1 to 8, **characterized in that** it includes means (38, 39) for holding the walls of the sleeve in their channel-forming shape.

10. A pouch according to claim 9, **characterized in that** the sleeve (218) has two holder tongues (250A, 250B) carried respectively by the first and second walls (220A, 220B) of the sleeve on the side opposite from the opening of the pouch, each of the tongues (250A, 250B) presenting a free head (251A, 251B) that is separated from the wall that carries it by a blocking slot (252A, 252B).

11. A pouch according to any one of claims 1 to 10, **characterized in that** the sleeve (118) presents a segment (118A) inside the bag, which segment has spreader tabs (140A, 140B) formed in continuity respectively with each of the two walls of the sleeve and having free lateral ends (140'A, 140'B) suitable for spreading apart from each other in the utilization configuration of the pouch in order to space apart the opposite faces of the flexible bag (12).

12. A pouch according to any one of claims 1 to 11, **characterized in that**, in a region remote from the opening (12A), the bag (12) presents a line of weakness (15A) suitable for being broken in order to form a tear that extends into the inside space of the bag.
